# EUROPEAN PATENT SPECIFICATION

(11) **EP 3 763 350 B1**
(45) Date of publication and mention of the grant of the patent: **31.08.2022**
(21) Application number: 19185992.5
(22) Date of filing: 12.07.2019
(51) Int. Cl.: A61K 8/02, A61K 8/04, A61K 8/20, A61K 8/25, A61K 8/81, A61Q 5/00, A61Q 5/02, A61K 8/34, A61K 8/19, A61K 8/46

(54) **DRY SHAMPOO WITH LOW RESIDUE STAINING PROPERTIES**
TROCKENSHAMPOO MIT GERINGEN RESTFÄRBUNGSEIGENSCHAFTEN
SHAMPOOING SEC AYANT DES PROPRIÉTÉS DE COLORATION À FAIBLES RÉSIDUS

(43) Date of publication of application: 13.01.2021
(73) Proprietor: KAO CORPORATION, Tokyo 103-8210 (JP)
(72) Inventor: Schmid, Sabine, 64297 Darmstadt (DE); Sulzbach, Melina, 64297 Darmstadt (DE)
(74) Representative: Hoffmann Eitle

(56) References cited:
- EP-A1- 2 090 295
- WO-A1-2015/059170
- WO-A2-2009/153311
- WO-A2-2014/095229
- US-A1- 2010 285 076

## Description

### Field of the invention

The present invention is directed to an aqueous composition, which may be used as a dry shampoo. The invention is also directed to a non-aerosol vaporizer, an aerosol vaporizer, a method for cleansing keratin fibers, and a kit-of-parts.

### Background of the invention

Dry shampoos are popular products to refresh the look of keratin fibers between two washes, thereby extending the time period for leaving the fibers non-washed. Many consumers with busy life-styles appreciate these types of products, as they are beneficial for use during travel, or directly prior to business appointments to polish the hair look. Moreover, dry shampoos have a niche application in regions with scarce water supplies, e.g. in dry regions of the planet, during military operations, or on the international space station.

All of the consumers mentioned above desire to use dry shampoo compositions which effectively clean the keratin fibers and leave no residues.

WO2015/059170 relates to a dry shampoo composition comprising a base and propellant, wherein the base comprises sebum absorber particles, said particles comprising acrylate cross-polymer, polydivinyl benzene polymers or hydrophobically modified polysaccharide derivatives, wherein the particles are insoluble in water.

WO2014095229 discloses a dry shampoo that simultaneously styles the hair. However, the disclosure is silent on insoluble particulates as in the present invention. Mintel 5593587 discloses a dry shampoo lacking multivalent ions.

### Summary of the invention

The first object of the present invention is an aqueous cosmetic composition suitable for vaporization with a non-aerosol vaporizer or aerosol vaporizer comprising:
- one or more particulate(s) having an average particle size in the range of 10 µm to 250 µm being insoluble in water at 25°C at atmospheric pressure,
- one or more inorganic salt(s) being soluble in water at 25°C at atmospheric pressure, selected from salts providing divalent and/or trivalent cations upon dissolution in water,
- one or more monohydric and/or dihydric alcohol(s).

The second object of the present invention is a non-aerosol vaporizer comprising the composition as defined above.

The third object of the present invention is an aerosol vaporizer comprising the composition as defined above and one or more cosmetically acceptable propellant(s).

The fourth object of the present invention is a method for cleansing keratin fibers, preferably human keratin fibers, more preferably human hair, comprising the steps of:
a) providing a composition as defined above,
b) vaporizing the composition with a non-aerosol vaporizer as defined above or an aerosol vaporizer as defined above, and applying the composition onto keratin fibers,
c) leaving the composition onto keratin fibers and letting it dry,
d) mechanically swirling and/or combing the keratin fibers.

Another object of the present invention is a kit-of-parts comprising the non-aerosol vaporizer as defined above or the aerosol vaporizer as defined above and a comb.

### Detailed description of the invention

Despite the attempts of the prior art, there is a great need to improve the cleansing performance of dry shampoos while not leaving staining residues on keratin fibers. The cleansing performance is important, because consumers desire to remove sebum and dirt from their hair. Additionally, the hair is supposed to look refreshed without having a grey staining. Moreover, upon application, smooth touch of the hair and good volume are important factors for the consumer.

The present invention addresses the problems mentioned above.

### Aqueous cosmetic composition

The present invention is directed to an aqueous cosmetic composition suitable for vaporization with a non-aerosol vaporizer or aerosol vaporizer comprising:
- one or more particulate(s) having an average particle size in the range of 10 µm to 250 µm being insoluble in water at 25°C at atmospheric pressure,
- one or more inorganic salt(s) being soluble in water at 25°C at atmospheric pressure, selected from salts providing divalent and/or trivalent cations upon dissolution in water,
- one or more monohydric and/or dihydric alcohol(s).

Preferably, the composition comprises less than 0.5% by weight of styling polymers, more preferably it is free of styling polymers, from the viewpoint of removability of the composition from keratin fibers.

Styling polymers within the meaning of the present invention are polymers, which dissolve in the aqueous-alcoholic composition of the present invention. Polymers and/or polymer-based particulates, which do not dissolve in the aqueous-alcoholic composition of the present invention, are considered as insoluble particulates.

### Insoluble particulates

The composition of the present invention comprises one or more particulate(s) having an average particle size in the range of 10 µm to 250 µm being insoluble in water at 25°C at atmospheric pressure.

Preferably, the one or more particulate(s) are inorganic particulate(s), from the viewpoint of residue removability.

Suitable one or more inorganic particulates are silica, preferably amorphous silica, mica, black iron oxide, carbon black, black titanium oxide, red iron oxide, yellow iron hydroxide, titanium iron oxide, titanium dioxide, manganese violet, ultramarine blue, chromium oxides, hydrated chromium oxides, ferric blue, zirconium oxide, bismuth oxychloride, bismuth citrate, zinc oxide, cerium oxide, aluminium, alimunium oxide hydrate, aluminium silicate, barium sulfate, calcium carbonate, calcium sulfate, cobalt aluminium oxide, copper powder, gold powder, Prussion blue, magnesium carbonate, manganese (II) phosphate, bronze powder, silver powder, ferric ammonium ferrocyanide, ferric ferrocyanide, henna, zinc sulphide, and/or their mixtures.

Preferably, the one or more particulate(s) are organic particulate(s), from the viewpoint of residue removability.

Suitable one or more organic particulates are polyvinylpyrrolidone-based particulates, such as commercially available under the trade name advantage revive polymer from Ashland Inc.

It is preferred from the viewpoint of residue removability and non-dyeing effect that one or more particulate is selected from silica, more preferably it is amorphous silica, and/or polyvinylpyrrolidone-based particulates, and/or their mixtures.

Particle sizes of the insoluble particulates are measured in powder form with static light scattering equipment using the volume-based particle size equation method. A suitable instrument for this measurement is the Malvern Mastersizer static light scattering instrument equipped with a powder measurement cell. For non-spherical particles an equivalent diameter is retrieved by this measurement method and the particle sizes apply to the equivalent diameters for non-spherical pigments.

It is preferred from the viewpoint of residue removability and cosmetic safety that the average particle size of the one or more particulate(s) is 10 µm or more.

It is further preferred from the viewpoint of residue removability that the average particle size of the one or more particulate(s) is 50 µm or less, more preferably 20 µm or less.

For attaining the above-mentioned effects, the preferred average particle size of the one or more particulate(s) is in the range of 10 µm to 50 µm, more preferably in the range of 10 µm to 20 µm.

Preferably, the total concentration of one or more particulates is 0.25% by weight or more, more preferably 0.5% by weight or more, further more preferably 1% by weight or more, still further more preferably 2% by weight or more, calculated to the total weight of the composition, from the viewpoint of residue removability, non-staining, and cleansing effect.

Preferably, the total concentration of one or more particulates is 15% by weight or less, more preferably 10% by weight or less, further more preferably 8% by weight or less, still further more preferably 6% by weight or less, calculated to the total weight of the composition, from the viewpoint of residue removability, non-staining, and cleansing effect.

For attaining the above-mentioned effects, the total concentration of one or more particulates is in the range of 0.25% to 15% by weight, preferably 0.5% to 10% by weight, more preferably 1% to 8% by weight, further more preferably 2% to 6% by weight, calculated to the total weight of the composition.

### Inorganic salt(s)

The composition of the present invention comprises one or more inorganic salt(s) being soluble in water at 25°C at atmospheric pressure, selected from salts providing divalent and/or trivalent cations upon dissolution in water.

Preferably, the one or more inorganic salt(s) is selected from magnesium salt(s), calcium salt(s), and/or aluminium salt(s), and/or their mixtures, preferably it is a magnesium salt(s), from the viewpoint of cosmetic safety, residue removability, and cost of goods.

Suitable salts are magnesium sulfate, calcium sufate, and aluminium chloride.

It is further preferred from the viewpoint of cosmetic safety, residue removability, cost of goods, and solubility that the one or more inorganic salt(s) is magnesium sulfate.

Preferably, the total concentration of one or more inorganic salt(s) is 0.25% by weight or more, more preferably 0.5% by weight or more, further more preferably 1% by weight or more, still further more preferably 2% by weight or more, calculated to the total weight of the composition, from the viewpoint of residue removability.

Preferably, the total concentration of one or more inorganic salt(s) is 15% by weight or less, more preferably 10% by weight or less, further more preferably 8% by weight or less, still further more preferably 6% by weight or less, calculated to the total weight of the composition, from the viewpoint solubility and cosmetic safety.

For attaining the above-mentioned effects, the total concentration of one or more inorganic salt(s) is 0.25% to 15% by weight, preferably 0.5% to 10% by weight, more preferably 1% to 8% by weight, further more preferably 2% to 6% by weight, calculated to the total weight of the composition.

### Monohydric/dihydric alcohol(s)

The composition of the present invention comprises one or more monohydric and/or dihydric alcohol(s).

Preferably, one or more monohydric and/or dihydric alcohol(s) is/are selected from ethanol, ethylene glycol, propylene glycol, and/or butylene glycol, and/or their mixtures, preferably it is ethanol, from the viewpoint of cosmetic safety and quick drying.

It is preferred that the total concentration of one or more monohydric and/or dihydric alcohol(s) is 0.5% by weight or more, more preferably 1% by weight or more, further more preferably 2% by weight or more, still further more preferably 4% by weight or more, calculated to the total weight of the composition, from the viewpoint of quick drying and residue removability.

It is preferred that the total concentration of one or more monohydric and/or dihydric alcohol(s) is 25% by weight or less, more preferably 20% by weight or less, further more preferably 15% by weight or less, still further more preferably 12% by weight or less, calculated to the total weight of the composition, from the viewpoint of residue removability and cosmetic safety.

For attaining the above-mentioned effects, the total concentration of one or more monohydric and/or dihydric alcohol(s) is in the range of 0.5% to 25% by weight, preferably 1% to 20% by weight, more preferably 2% to 15% by weight, further more preferably 4% to 12% by weight, calculated to the total weight of the composition.

### Surfactants

The composition of the present invention may comprise one or more surfactant(s), preferably selected from non-ionic, anionic, and/or cationic surfactant(s), and/or their mixtures, from the viewpoint of increasing wettability of keratin fibers and insoluble particulates.

Suitable non-ionic surfactants may be selected from alkyl polyglycosides, ethoxylated triglycerides, ethoxylated fatty alcohols, ethoxylated fatty acid esters, and/or their mixtures.

Preferably, the anionic surfactants may be selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures.

Suitable examples are alkyl sulfate or preferably ethoxylated alkyl ether sulfate surfactant or mixtures thereof having an alkyl chain length of C₁₀ to C₂₂.

Suitable cationic surfactants are quaternary ammonium surfactants having a carbon chain length in the range of C₁₂ to C₂₂ or surfactants having a tertiary amine group and at least one alkyl chain having a carbon chain length in the range of C₁₂ to C₂₂ such as alkylamidoalkylamine surfactants. Suitable examples are cetrimonium chloride.

Suitable concentration ranges for surfactants are in the range of 0.1% to 2% by weight, calculated to the total weight of the composition, from the viewpoint of enhancing wettability of keratin fibers and insoluble particulates.

### Non-aerosol vaporizer

The present invention is also directed to a non-aerosol vaporizer comprising the composition as defined above.

Suitable non-aerosol vaporizers are trigger sprays and pump spray assemblies. Such trigger sprays are, for example, disclosed in JP2008200572.

Trigger sprays are liquid sprayers provided with a sprayer main body and a spray nozzle at the tip while incorporating a pump mechanism and a cap for fixing and holding the main body at a spout portion of a container. Additionally, they have an operation lever for actuating the pump by pulling operation of the lever to spray the liquid from the container through the spray nozzle.

Pump spray assemblies are, for example, disclosed in JP2011230842. Pump sprays eject a liquid stream by pushing down a spray head, and in which the spray head is attached on a pump for pumping the liquid and a pushing down operation is performed with a pressing member that moves the spray head up and down.

### Aerosol vaporizer

The present invention is also directed to an aerosol vaporizer comprising the composition as defined above and one or more cosmetically acceptable propellant(s).

Suitably, conventional spray cans may be used for that purpose. The composition of the present invention is filled in the spray can, the can is then closed, and a suitable propellant is introduced into the spray can. Then the nozzle is attached to the can and the user may actuate the nozzle by a pressing member to vaporize the composition.

Suitable propellants are carbon dioxide, dimethylether, n-butane, iso-butane, n-pentane, n-hexane, liquefied petroleum gas, hydrofluorcarbon gases, and/or their mixtures.

The composition may comprise propellants at a total concentration of 30% to 90% by weight, preferably 45% to 85% by weight, more preferably 60% to 80% by weight, calculated to the total weight of the aerosol composition.

Depending on the type of aerosol can and type of propellant used, the pressure inside the aerosol can is usually kept below 12 bar, preferably it is around 8.5 bar, further preferably it is below 5 bar, from the viewpoint of cosmetic safety.

### Method for cleansing

The present invention also is directed to a method for cleansing keratin fibers, preferably human keratin fibers, more preferably human hair comprising the steps of:
a) providing a composition as defined above,
b) vaporizing the composition with a non-aerosol vaporizer as defined above or an aerosol vaporizer as defined above, and applying the composition onto keratin fibers,
c) leaving the composition onto keratin fibers and letting them dry,
d) mechanically swirling and/or combing the keratin fibers.

Preferably, from the viewpoint of cleansing efficiency and refreshing look, about 0.2 g of composition is applied to 2 g of keratin fibers. Thus, the preferable weight ratio of composition to keratin fibers is in the range of 1:10 to 1:5, from the viewpoint of cleansing efficiency.

Preferably, the drying step of c) is an air-drying step from the viewpoint of environmental considerations. Alternatively, step c) may also be a blow-drying step from the viewpoint of drying speed. Thus, a preferably time range of leaving the composition onto keratin fibers is between 10 s and 600 s, from the viewpoint of drying the fibers prior to combing.

The mechanically swirling of step d) refreshes the hair look and removes coarse dirt or sebum particles. The combing step of d) removes sebum and dirt, and, if necessary, any product residues. Therefore, combing is preferably conducted from roots to lengths from the viewpoint of cleansing efficiency.

### Kit-of-parts

The present invention is also directed to a kit-of-parts comprising the non-aerosol vaporizer as defined above or the aerosol vaporizer as defined above and a comb.

It is to be noted that this kit is directly applicable for sale to professional hair dressers and end consumers alike.

The following examples are to illustrate the invention, but not to limit it.

### EXAMPLES

An alcohol/water mixture was prepared and then the soluble salts were dissolved in this mixture. Finally, the insoluble particles were added. The composition was filled into a commercially available pump spray, shaken directly prior to vaporizing, and then sprayed onto human hair. About 0.2 g of composition was applied to about 2 g of human hair.

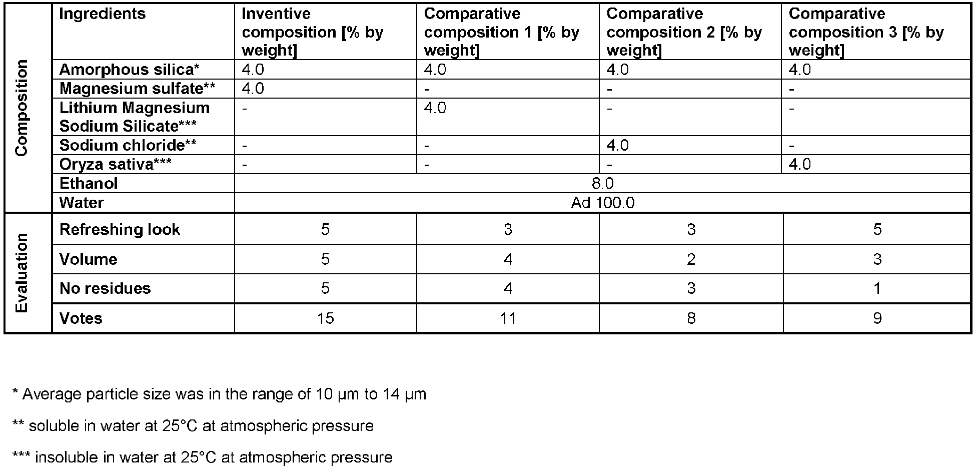

### Methods

### Sensory evaluation

Evaluation of compositions was conducted on hair of 10 human volunteers by well-trained hair dressers who are specialized in the field of hair care. A half-head comparison test was performed by applying the inventive composition on one side of the head and one of the comparative composition onto the other side. The compositions were allowed to air-dry and the volunteer's hair was combed for 2 min per side with a standard professional comb. Then 5 other hair-dresser colleagues were asked to independently rate each side without them knowing which side was treated with inventive or comparative composition. The colleagues were asked to rate with integer numbers between 1 and 5 for each category (refreshing look, volume, no residues).

The numbers denoted as follows:
5 very good
4 good
3 medium
2 poor
1 very poor

The arithmetic mean of the integer numbers were reported above and mathematically rounded.

### Particle size measurement

Particle sizes were measured in powder form with static light scattering equipment using the volume-based particle size equation method. A suitable instrument for this measurement is the Malvern Mastersizer static light scattering instrument equipped with a powder measurement cell. For non-spherical particles an equivalent diameter is retrieved by this measurement method and the particle sizes apply to the equivalent diameters for non-spherical pigments.

The following examples are within the scope of the present invention.

| **Inventive example 2** | |
|---|---|
| | **% by weight** |
| Amorphous silica | 2.0 |
| Magnesium sulfate | 6.0 |
| Ethanol | 5.0 |
| Ceteareth-20 | 0.5 |
| Fragrance | 0.3 |
| Water | Ad 100.0 |

| **Inventive example 3** | |
|---|---|
| | **% by weight** |
| Amorphous silica | 2.0 |
| Calcium sulfate | 2.0 |
| Ethanol | 1.0 |
| Butylene glycol | 0.5 |
| Fragrance | 0.3 |
| Water | Ad 100.0 |

| **Inventive example 4** | |
|---|---|
| | % **by weight** |
| PVP particulates* | 2.0 |
| Amorphous silica | 2.0 |
| Magnesium sulfate | 2.0 |
| Ethanol | 1.0 |
| Fragrance | 0.3 |
| Water | Ad 100.0 |

| | |
|---|---|
| * PVP particulates sold under trade name Advantage Revive Polymer by Ashland Inc. | |

| **Inventive example 4** | |
|---|---|
| | % **by weight** |
| PVP particulates* | 6.0 |
| Magnesium sulfate | 6.0 |
| Ethanol | 3.0 |
| Fragrance | 0.3 |
| Water | Ad 100.0 |

| | |
|---|---|
| * PVP particulates sold under trade name Advantage Revive Polymer by Ashland Inc. | |

## Claims

1. An aqueous cosmetic composition suitable for vaporization with a non-aerosol vaporizer or aerosol vaporizer, comprising:
- one or more particulate(s) having an average particle size in the range of 10 µm to 250 µm being insoluble in water at 25°C at atmospheric pressure,
- one or more inorganic salt(s) being soluble in water at 25°C at atmospheric pressure, selected from salts providing divalent and/or trivalent cations upon dissolution in water,
- one or more monohydric and/or dihydric alcohol(s).

2. The composition according to claim 1 **characterized in that** it comprises less than 0.5% by weight of styling polymers, preferably it is free of styling polymers.

3. The composition according to claims 1 and/or 2 **characterized in that** one or more particulate is selected from silica, preferably it is amorphous silica, and/or polyvinylpyrrolidone-based particulates, and/or their mixtures.

4. The composition according to any of the preceding claims **characterized in that** the average particle size of the one or more particulate(s) is in the range of 10 µm to 50 µm, more preferably in the range of 10 µm to 20 µm.

5. The composition according to any of the preceding claims **characterized in that** the total concentration of one or more particulates is in the range of 0.25% to 15% by weight, preferably 0.5% to 10% by weight, more preferably 1% to 8% by weight, further more preferably 2% to 6% by weight, calculated to the total weight of the composition.

6. The composition according to any of the preceding claims **characterized in that** the one or more inorganic salt(s) is selected from magnesium salt(s), calcium salt(s), and/or aluminium salt(s), and/or their mixtures, preferably it is a magnesium salt(s).

7. The composition according to any of the preceding claims **characterized in that** the one or more inorganic salt(s) is magnesium sulfate.

8. The composition according to any of the preceding claims **characterized in that** the total concentration of one or more inorganic salt(s) is 0.25% to 15% by weight, preferably 0.5% to 10% by weight, more preferably 1% to 8% by weight, further more preferably 2% to 6% by weight, calculated to the total weight of the composition.

9. The composition according to any of the preceding claims **characterized in that** the one or more monohydric and/or dihydric alcohol(s) are selected from ethanol, ethylene glycol, propylene glycol, and/or butylene glycol, and/or their mixtures, preferably it is ethanol.

10. The composition according to any of the preceding claims **characterized in that** the total concentration of one or more monohydric and/or dihydric alcohol(s) is in the range of 0.5% to 25% by weight, preferably 1% to 20% by weight, more preferably 2% to 15% by weight, further more preferably 4% to 12% by weight, calculated to the total weight of the composition.

11. The composition according to any of the preceding claims **characterized in that** it comprises one or more surfactant(s), preferably selected from non-ionic, anionic, and/or cationic surfactant(s), and/or their mixtures.

12. A non-aerosol vaporizer comprising the composition as defined in the claims 1 to 11.

13. An aerosol vaporizer comprising the composition as defined in the claims 1 to 11 and one or more cosmetically acceptable propellant(s).

14. A method for cleansing keratin fibers, preferably human keratin fibers, more preferably human hair, comprising the steps of:
a) providing a composition as defined in claims 1 to 11,
b) vaporizing the composition with a non-aerosol vaporizer as defined in claim 12 or an aerosol vaporizer as defined in claim 13, and applying the composition onto keratin fibers,
c) leaving the composition onto keratin fibers and letting them dry,
d) mechanically swirling and/or combing the keratin fibers.

15. Kit-of-parts comprising the non-aerosol vaporizer as defined in claim 12 or the aerosol vaporizer as defined in claim 13 and a comb.

## Patentansprüche

1. Wässrige kosmetische Zusammensetzung, geeignet zum Verdampfen mit einem Nicht-Aerosol-Verdampfer oder Aerosol-Verdampfer, umfassend:
- einen oder mehrere Partikel mit einer durchschnittlichen Partikelgröße im Bereich von 10 µm bis 250 µm, die bei 25°C und atmosphärischem Druck in Wasser unlöslich sind,
- ein oder mehrere anorganische(s) Salz(e), löslich bei 25°C und atmosphärischem Druck in Wasser, ausgewählt aus Salzen, die bei dem Auflösen in Wasser zweiwertige und/oder dreiwertige Kationen bilden,
- einen oder mehrere ein- und/oder zweiwertige(n) Alkohol(e).

2. Zusammensetzung gemäß Anspruch 1, **dadurch gekennzeichnet, dass** sie weniger als 0,5 Gew.-% Stylingpolymere umfasst, vorzugsweise ist sie frei von Stylingpolymeren.

3. Zusammensetzung gemäß Anspruch 1 und/oder 2, **dadurch gekennzeichnet, dass** der eine oder die mehreren Partikel aus Silica, vorzugsweise ist es amorphes Silica, und/oder Polyvinylpyrrolidon-basierten Partikeln und/oder deren Mischungen ausgewählt ist/sind.

4. Zusammensetzung gemäß einem der vorhergehenden Ansprüche, **dadurch gekennzeichnet, dass** die durchschnittliche Partikelgröße des einen oder der mehreren Partikel im Bereich von 10 µm bis 50 µm, vorzugsweise im Bereich von 10 µm bis 20 µm, liegt.

5. Zusammensetzung gemäß einem der vorangehenden Ansprüche, **dadurch gekennzeichnet, dass** die Gesamtkonzentration des einen oder der mehreren Partikel(s) im Bereich von 0,25 bis 15 Gew.-%, bevorzugt 0,5 bis 10 Gew.-%, mehr bevorzugt 1 bis 8 Gew.-%, noch mehr bevorzugt 2 bis 6 Gew.-%, berechnet auf das Gesamtgewicht der Zusammensetzung, liegt.

6. Zusammensetzung gemäß einem der vorhergehenden Ansprüche, **dadurch gekennzeichnet, dass** das eine oder die mehreren anorganische(n) Salz(e) aus Magnesiumsalz(en), Calciumsalz(en) und/oder Aluminiumsalz(en) und/oder deren Mischungen ausgewählt ist/sind, vorzugsweise ist es ein Magnesiumsalz(e).

7. Zusammensetzung gemäß einem der vorhergehenden Ansprüche, **dadurch gekennzeichnet, dass** das eine oder die mehreren anorganische(n) Salz(e) Magnesiumsulfat ist.

8. Zusammensetzung gemäß einem der vorhergehenden Ansprüche, **dadurch gekennzeichnet, dass** die Gesamtkonzentration des einen oder der mehreren anorganischen Salze(s) 0,25 bis 15 Gew.-%, bevorzugt 0,5 bis 10 Gew.-%, mehr bevorzugt 1 bis 8 Gew.-%, noch mehr bevorzugt 2 bis 6 Gew.-%, berechnet auf das Gesamtgewicht der Zusammensetzung, beträgt.

9. Zusammensetzung gemäß einem der vorhergehenden Ansprüche, **dadurch gekennzeichnet, dass** der eine oder die mehreren ein- und/oder zweiwertige(n) Alkohol(e) aus Ethanol, Ethylenglycol, Propylenglycol und/oder Butylenglycol und/oder deren Mischungen ausgewählt ist/sind, vorzugsweise ist es Ethanol.

10. Zusammensetzung gemäß einem der vorhergehenden Ansprüche, **dadurch gekennzeichnet, dass** die Gesamtkonzentration des einen oder der mehreren ein-und/oder zweiwertigen Alkohole im Bereich von 0,5 bis 25 Gew.-%, bevorzugt 1 bis 20 Gew.-%, mehr bevorzugt 2 bis 15 Gew.-%, noch mehr bevorzugt 4 bis 12 Gew.-%, berechnet auf das Gesamtgewicht der Zusammensetzung, liegt.

11. Zusammensetzung gemäß einem der vorhergehenden Ansprüche, **dadurch gekennzeichnet, dass** sie ein oder mehrere Tensid(e), vorzugsweise ausgewählt aus nichtionischen, anionischen und/oder kationischen Tensiden und/oder deren Mischungen, enthält.

12. Nicht-Aerosol-Verdampfer, umfassend die Zusammensetzung, wie in den Ansprüchen 1 bis 11 definiert.

13. Aerosolverdampfer, umfassend die Zusammensetzung, wie in den Ansprüchen 1 bis 11 definiert, und ein oder mehrere kosmetisch verträgliche(s) Treibmittel.

14. Verfahren zur Reinigung von Keratinfasern, bevorzugt menschlichen Keratinfasern, mehr bevorzugt menschlichem Haar, umfassend die Schritte:
a) das Bereitstellen einer Zusammensetzung, wie in den Ansprüchen 1 bis 11 definiert,
b) das Verdampfen der Zusammensetzung mit einem Nicht-Aerosol-Verdampfer, wie in Anspruch 12 definiert, oder einem Aerosol-Verdampfer, wie in Anspruch 13 definiert, und das Auftragen der Zusammensetzung auf die Keratinfasern,
c) das Belassen der Zusammensetzung auf den Keratinfasern und das Trocknenlassen derselben,
d) das mechanische Verwirbeln und/oder Kämmen der Keratinfasern.

15. Teilesatz, umfassend den Nicht-Aerosol-Verdampfer, wie in Anspruch 12 definiert, oder den Aerosol-Verdampfer wie in Anspruch 13 definiert, und einen Kamm.

## Revendications

1. Composition cosmétique aqueuse adéquate pour une pulvérisation avec un pulvérisateur non aérosol ou un pulvérisateur aérosol, comprenant :
- une ou plusieurs matières particulaires présentant une taille de particule moyenne dans la plage de 10 pm à 250 pm qui est insoluble dans l'eau à 25 °C à la pression atmosphérique,
- un ou plusieurs sels inorganiques qui sont solubles dans l'eau à 25 °C à la pression atmosphérique, sélectionnés parmi des sels fournissant des cations divalents et/ou trivalents lors d'une dissolution dans l'eau,
- un ou plusieur alcools monohydriques et/ou dihydriques.

2. Composition selon la revendication 1, **caractérisée en ce qu'**elle comprend moins de 0,5 % en poids de polymères coiffants, de préférence elle est exempte de polymères coiffants.

3. Composition selon les revendications 1 et/ou 2, **caractérisée en ce qu'**une ou plusieurs matières particulaires sont sélectionnées parmi la silice, de préférence il s'agit d'une silice amorphe, et/ou des matières particulaires à base de polyvinylpyrrolidone et/ou leurs mélanges.

4. Composition selon l'une quelconque des revendications précédentes, **caractérisée en ce que** la taille de particule moyenne des une ou plusieurs matières particulaires se situe dans la plage de 10 pm à 50 pm, plus préférentiellement dans la plage de 10 µm à 20 µm.

5. Composition selon l'une quelconque des revendications précédentes, **caractérisée en ce que** la concentration totale d'une ou plusieurs matières particulaires se situe dans la plage de 0,25 % à 15 % en poids, de préférence de 0,5 % à 10 % en poids, plus préférentiellement de 1% à 8 % en poids, encore plus préférentiellement de 2 % à 6 % en poids, calculée par rapport au poids total de la composition.

6. Composition selon l'une quelconque des revendications précédentes, **caractérisée en ce que** les un ou plusieurs sels inorganiques sont sélectionnés parmi un ou plusieurs sels de magnésium, un ou plusieurs sels de calcium et/ou un ou plusieurs sels d'aluminium et/ou leurs mélanges, de préférence il s'agit d'un ou plusieurs sels de magnésium.

7. Composition selon l'une quelconque des revendications précédentes, **caractérisée en ce que** les un ou plusieurs sels inorganiques sont le sulfate de magnésium.

8. Composition selon l'une quelconque des revendications précédentes, **caractérisée en ce que** la concentration totale d'un ou plusieurs sels inorganiques est de 0,25 % à 15 % en poids, de préférence de 0,5 % à 10% en poids, plus préférablement de 1% à 8 % en poids, encore plus préférentiellement de 2 % à 6 % en poids, calculée sur la base du poids total de la composition.

9. Composition selon l'une quelconque des revendications précédentes, **caractérisée en ce que** les un ou plusieurs alcools monohydriques et/ou dihydriques sont sélectionnés parmi l'éthanol, l'éthylène glycol, le polypropylène glycol et/ou le butylène glycol et/ou leurs mélanges, de préférence il s'agit de l'éthanol.

10. Composition selon l'une quelconque des revendications précédentes, **caractérisée en ce que** la concentration totale d'un ou plusieurs alcools monohydriques et/ou dihydriques se situe dans la plage de 0,5 % à 25 % en poids, de préférence de 1 % à 20 % en poids, plus préférentiellement de 2 % à 15 % en poids, encore plus préférentiellement de 4 % à 12 % en poids, calculée sur la base du poids total de la composition.

11. Composition selon l'une quelconque des revendications précédentes, **caractérisée en ce qu'**elle comprend un ou plusieurs tensioactifs, de préférence sélectionnés parmi un ou plusieurs tensioactifs non ioniques, anioniques et/ou cationiques et/ou leurs mélanges.

12. Pulvérisateur non aérosol comprenant la composition selon les revendications 1 à 11.

13. Pulvérisateur aérosol comprenant la composition selon les revendications 1 à 11 et un ou plusieurs propulseurs cosmétiquement acceptables.

14. Procédé pour nettoyer des fibres de kératine, de préférence des fibres de kératine humaine, plus préférentiellement des cheveux humains, comprenant les étapes consistant à :
a) fournir une composition selon les revendications 1 à 11,
b) pulvériser la composition avec un pulvérisateur non aérosol selon la revendication 12 ou un pulvérisateur aérosol selon la revendication 13 et appliquer la composition sur des fibres de kératine,
c) laisser la composition sur des fibres de kératine et laisser sécher,
d) faire tournoyer mécaniquement et/ou peigner les fibres de kératine,

15. Kit de pièces comprenant le pulvérisateur non aérosol selon la revendication 12 ou le pulvérisateur aérosol selon la revendication 13 et un peigne.
